# EUROPEAN PATENT SPECIFICATION

(11) **EP 2 862 575 B1**
(45) Date of publication and mention of the grant of the patent: **27.03.2019**
(21) Application number: 13807436.4
(22) Date of filing: 19.06.2013
(51) Int. Cl.: A61K 31/7034, A61P 9/00, A61P 9/10, A61K 47/10, A61K 47/26, A61K 47/40, A61K 9/00, A61K 31/724

(54) **APPLICATION OF PICEATANNOL-3'-O-B-D-GLUCOPYRANOSIDE IN PREPARATION OF MEDICAMENTS FOR IMPROVING MICROCIRCULATION BLOCK**
ANWENDUNG VON PICEATANNOL-3'-O-B-D-GLUCOPYRANOSID BEI DER HERSTELLUNG VON MEDIKAMENTEN ZUR VERBESSERUNG EINER MIKROZIRKULATIONSSPERRE
APPLICATION DE PICÉATANNOL-3'-O-B-D-GLUCOPYRANOSIDE DANS LA PRÉPARATION DE MÉDICAMENTS POUR L'AMÉLIORATION DU BLOCAGE DE LA MICROCIRCULATION

(30) Priority: 19.06.2012 CN 201210202957
(43) Date of publication of application: 22.04.2015
(73) Proprietor: KPC Pharmaceuticals, Inc., Kunming, Yunnan 650106 (CN)
(72) Inventor: CHEN, Yunjian, Kunming Yunnan 650106 (CN); LIU, Yidan, Kunming Yunnan 650106 (CN); ZHU, Ze, Kunming Yunnan 650106 (CN); YANG, Zhaoxiang, Kunming Yunnan 650106 (CN); SHANG, Jianhua, Kunming Yunnan 650106 (CN); YANG, Xujuan, Kunming Yunnan 650106 (CN)
(74) Representative: HGF Limited
(86) International application number: PCT/CN2013/077442
(87) International publication number: WO 2013/189285

(56) References cited:
- EP-A1- 2 832 348
- WO-A1-2004/032941
- CN-A- 1 294 912
- CN-A- 101 787 061
- CN-A- 102 058 679
- CN-A- 102 276 666
- CN-A- 103 417 556
- JP-A- 2007 223 919
- KR-A- 20110 112 783
- WOO, AINIENG ET AL.: 'Piceatannol-3'-O-[3-D-glucopyranoside as an active component of rhubarb activates endothelial nitric oxide synthase through inhibition of arginase activity' EXPERIMENTAL AND MOLECULAR MEDICINE vol. 42, no. 7, 14 June 2010, pages 524 - 532, XP055180631

## Description

### FIELD OF THE INVENTION

The present invention belongs to the field of pharmaceutical chemistry, and particularly to the application of piceatannol-3'-o-β-d-glucopyranoside in a pharmaceutical composition for use in improving microcirculation block.

### BACKGROUND OF THE INVENTION

Microcirculation refers to the blood circulation between arteriole and venule. The fundamental function of microcirculation is based on the mass exchange between blood and tissue fluid. Under normal conditions, the blood flow volume involved in microcirculation is compatible with the metabolic level in tissues or organs, in order to maintain the blood perfusion volume in each tissue or organ and to regulate the returned blood volume. If there is a disorder occurred in microcirculation, the physiological functions in each organ will be influenced directly. Smooth muscle is mainly contained in arteriole, metarteriole, precapillary sphincter and venule, and its relaxation and contraction will direct influence the blood flow volume of microcirculation. The contraction of arteriole and venule as well as the reduction of the open number of capillary vessel net due to diseases will block the regular blood flow in microcirculation, resulting in lesions due to tissue ischemia.

Microcirculation block refers to a series of clinical symptoms resulted from local ischemia or hypoxia or even necrosis in tissues, due to stenosis, slow blood flow rate or thrombosis, which may be resulted from the variation of blood constituents or vasculopathy. If microthrombus is formed due to the adhesion among thrombocyte, leucocyte or fibrin in microcirculation and flows to each part of the body with blood or deposits on intima, a series of symptoms will occur due to local blood block by thrombus, by which corresponding organization systems or internal organs will be influenced, so that normal functions can not performed, leading to senescence, disorder of immunologic functions and diseases. It has been suggested that microcirculation improvement plays a favorable role in prophylaxis and treatment of transient cerebral ischemia. The improvement of microcirculation is closely related to thrombolysis, toxicity of antioxidative or contrastimulant amino acid, resistance to overload of calcium, anti-inflammation, and anti-apoptosis. It has been described in "Plain Questions: the theory for the generation of the five internal organs" that "people can see by eye due to the blood flow, people can walk on foot due to the blood flow, people can hold by hand due to the blood flow, and people can move their fingers due to the blood flow", indicating that normal functions can be performed only when healthy blood supply is provided to organs. As the saying goes: "when the microcirculation is not hindered, stroke will not occur; when the microcirculation is good, coronary occlusion will less occur; when the microcirculation is smooth, people are healthy". Microcirculation is almost the reason for every disease, and favorable function of microcirculation is a primary prerequisite for normal functions of important organs. It has been demonstrated by modern medical science that many diseases, including senescence, tumors, hypertension, diabetes and various cardiovascular and cerebrovascular diseases, are resulted from microcirculation block, so that whether the microcirculation is normal or not can be an important sign for human health.

At present, there are medical and non-medical methods for the treatment of microcirculation block in clinic. The medicines mainly includes: vascular protectant for microcirculation, such as calcium 2,5-dihydroxyphenyl sulfonate, butylphthalide, and Difrarel etc.; anticoagulants, such as heparin, Fraxiparine, and warfarin; drugs for thrombolysis and anti-fibrinogen, such as streptokinase, urokinase, and tissue-type plasminogen activator etc.; antiplatelet drugs, such as aspirin, persantine, and ticlopidine etc.; vasodilator, such as trental, cinepazide maleate, kallidinogenase etc.; drugs for lowering blood viscosity, such as low molecular weight dextran, lovastatin, and simvastatin etc.; and formulations of Chinese traditional medicine, such as Di'ao Xinxuekang, Buchang Naoxintong, Compound Danshen Dripping Pills and Xueshuantong etc. Most medicines, no matter Western medicines or Chinese medicines, only target to the symptoms due to microcirculation block in specific part, such as the organs, for example, brain or heart. Although there are medicines for improving systemic symptoms due to microcirculation block, they have disadvantages, for example, attending to one thing and losing another, unobvious effects, or strong toxic and side effects. Since there is no symptom or unobvious symptom at the early stage of microcirculation block in most cases, it may be easily overlooked. However, the reason for microcirculation block will have potential impact on the whole microcirculatory system, and only the symptoms for the microcirculation block in certain parts are distinct, so that people may be treated only where the pain is. However, only the drug that is effective for the whole microcirculatory system is preferable for the treatment and prevention of microcirculation block.

Piceatannol-3'-o-β-d-glucopyranoside, with the chemical name of (E)-1-(3,5-dihydroxyphenyl)-2-(3-hydroxy-4-O-β-D-glucopyranose phenyl)ethylene or 3,5,3',4'-tetrahydroxy-stilbene-3'-O-β-glucoside (also as 3,5,4'-trihydroxy-stilbene-3'-O-glucoside) is named as stilbene-glucoside of Quzha, since it is derived from the root and stem of *Rheum lhasaense*, which is called as Quzha in Tibetan medicine ("New Revised Jingzhu Materia Medica, Luo Dashang (Ed.), Sichuan Science and Technology Press, 2004). It has been suggested by the investigation that piceatannol-3'-o-β-d-glucopyranoside has favorable safety, in which the single maximum dosage given by gastric gavage is 13.33 g/kg.bw or 5 g/kg.bw for mouse or beagle dog, respectively within 24 h in an acute toxicity test, and there is no toxic and side effect and death observed in animals. There is also no chromosome aberration or mutagenesis observed in genotoxicity test. In application No. 201010116358.2 titled "Application of piceatannol-3'-o-β-d-glucopyranoside in preparation of formulations for preventing and treating cardiac-cerebral ischemia diseases, and preparation method thereof', it is believed that the myocardial infarction can be reduced, the platelet aggregation can be inhibited, the myocardial ischemia can be protected and the cerebral infarction area can be decreased by piceatannol-3'-o-β-d-glucopyranoside; In application No. 201110166486.2, titled "Crystal of piceatannol-3'-o-β-d-glucopyranoside and the preparation method and application thereof", it has been suggested that the neurological dysfunction of rat can be improved and the cerebral infarction area can be decreased by piceatannol-3'-o-β-d-glucopyranoside, and it is also believed that piceatannol-3'-o-β-d-glucopyranoside has relatively strong radical scavenging activity, and can be applied in the prevention and treatment of cardiac-cerebral ischemia; in application No. 02134928.2, titled "Pharmaceutical composition containing polydatin or a salt thereof and application in pharmaceutical preparation", it is thought that polydatin or a salt thereof plays a role in the treatment and prevention of microcirculation block. Although there are reports on the applications of piceatannol-3'-o-β-d-glucopyranoside, its dissolution problems have not been solved in pharmaceutical formulation and in absorption process in human body, since the solubility of piceatannol-3'-o-β-d-glucopyranoside is poor in water. In the present invention, it has been confirmed that the solubility of piceatannol-3'-o-β-d-glucopyranoside can be greatly enhanced by gastrodin and/or water soluble cyclodextrin derivatives, which is helpful for the preparation of phamacuetical formulation and the absorption in human body.

It has been suggested by published literatures that stilbenes, including piceatannol-3'-o-β-d-glucopyranoside, possesses activities in anti-inflammation, anti-HIV, anti-tumor and treatment of cardiovascular and cerebrovascular diseases. Although there are some experimental conclusions about the prevention and treatment of cardiovascular and cerebrovascular diseases, the influence on microcirculatory system has not been described. Although application of polydatin or a salt thereof has been disclosed on the treatment and prevention of microcirculation block, there is no report on the use of piceatannol-3'-o-β-d-glucopyranoside in the medicines for microcirculation block other than the cardiovascular disorders.

Based on the disadvantages of the present medicines for microcirculation block, such as single function, poor curative effect and strong toxic and side effect, people now focus on discovery, research and development of ideal medicines for microcirculation block from phytomedicines in traditional medical science. Although the applications of polydatin or a salt thereof, Panax notoginseng saponins, ginsenoside Rb2 monomer, composition of calcium phenolsulfonate and pueraria flavonid, and composition of ginsenoside and breviscapine have been developed for the treatment of microcirculation block in recent years, there are also some disadvantages including unobvious clinical therapeutic effect, great side effect and uncontrollable addiction. If a compound with definite pharmacology, distinct therapeutic effect, wide indications, and no or little toxic and side effect can be found from Chinese traditional medicines, it will show the fascination of Chinese traditional medical science, and establish a new direction for the prevention and treatment of microcirculation block.

### SUMMARY OF THE INVENTION

The objective of the present invention is to provide novel applications of piceatannol-3'-o-β-d-glucopyranoside in pharmaceutical compositions for improving microcirculation block, the properties of which include: definite pharmacological action, distinct therapeutic effect, wide range of indications, and no or little toxic and side effect. Also disclosed is the preparation of medicaments for improving microcirculation block.

In accordance with claim 1, an aspect of the invention relates to a pharmaceutical formulation solution comprising piceatannol-3'-o-β-d-glucopyranoside and gastrodin for use in improving microcirculation block, wherein the piceatannol-3'-o-β-d-glucopyranoside is (E)-1-(3,5-dihydroxyphenyl)-2-(3-hydroxy-4-O-β-D-glucopyranose phenyl)ethylene of the following structure: 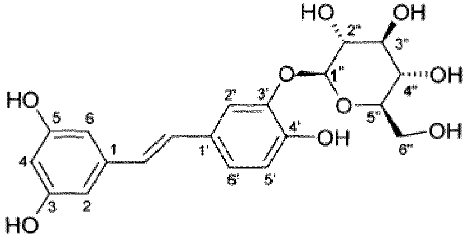 wherein the microcirculation block is selected from block induced by brain microvascular injury and/or block induced by ischemical reperfusion injury. Selected further features are set out in the dependent claims.

In prior art, there is no definite pharmacological action reported on the treatment of cerebrovascular diseases by piceatannol-3'-o-β-d-glucopyranoside, and there is also no report on the application of piceatannol-3'-o-β-d-glucopyranoside in the medicament for the treatment of microcirculation block except for cerebrovascular diseases. It has been indicated by the tests using Kunming normal mice and model mice with microcirculation block induced artificially that piceatannol-3'-o-β-d-glucopyranoside has obvious expansion effect on capillary vessels of the normal mice, can evidently antagonize the shrinkage of arteriole and venule and the reduction of the open number of capillary vessel net of the model mice with microcirculation block, and can obviously confront the blood flow slowing. It has thus fully described the pharmacological action of piceatannol-3'-o-β-d-glucopyranoside. In some patents, it has been confirmed that neurological dysfunction in rats can be improved and the range of cerebral infarction or acute myocardial infarction can be significantly reduced by piceatannol-3'-o-β-d-glucopyranoside; and the survival time can be significantly extended by piceatannol-3'-o-β-d-glucopyranoside in burn shock test of SD rat. There is no toxic effect observed when administrated in a range of safe dose in acute and long-term toxicity tests in mice. As a result, piceatannol-3'-o-β-d-glucopyranoside has the following properties in improving microcirculation block, including definite pharmacological action, distinct therapeutic effect, wide range of indications, and no or little toxic and side effect.

### DETAILED EMBODIMENTS

The present invention will be further described in the following sections. However, it should not be construed as limits of the invention, and any modifications based on the teachings of the present invention are all embraced in the scope of the present invention.

Piceatannol-3'-o-β-d-glucopyranoside has applications in preparation of medicaments for improving microcirculation block, and the piceatannol-3'-o-β-d-glucopyranoside is (E)-1-(3,5-dihydroxyphenyl)-2-(3-hydroxy-4-O-β-D-glucopyranose phenyl)ethylene.

Disclosed is a medicament for improving microcirculation block which is a formulation comprising piceatannol-3'-o-β-d-glucopyranoside and one or more of pharmaceutically acceptable additives.

As preferable embodiment, the content of piceatannol-3'-o-β-d-glucopyranoside is in the ratio from 1:0.1-80 by weight.

As preferable embodiment, the medicament for improving microcirculation block comprises water soluble cyclodextrin derivatives, or PEG400 and mannitol. Gastrodin and optionally water soluble cyclodextrin derivatives have solubilization and stabilization effects on piceatannol-3'-o-β-d-glucopyranoside.

Gastrodin is 4-hydroxymethylphenyl-β-D-glucopyranoside hemihydrate, which has a relatively stable structure of phenolic glycoside. Gastrodin is a white crystalline powder, which is odorless and bitter in taste; and its melting point is from 153°C to 156°C. Gastrodin is easily soluble in methanol, and soluble in ethanol. Gastrodin has the effects of sedation, hypnosis and abirritation, and has low toxicity. The maximum tolerated dose for human can be calculated as 33.249 g (on the basis of a body weight of 60 kg) based on the maximum tolerated dose of intramuscular injection and intravenous injection for animals. Gastrodin has the best water solubility in glycosides, which is as high as over 50%. However, the investigation of gastrodin has not been reported as a solubilizing agent.

As preferable embodiments, the water soluble cyclodextrin derivatives are selected from α-cyclodextrin derivatives, β-cyclodextrin derivatives and γ-cyclodextrin derivatives of various substitution degrees, or any combination thereof.

As further preferable embodiments, the water soluble cyclodextrin derivatives are selected from methyl-β-cyclodextrin, hydroxypropyl-β-cyclodextrin, thioether-β-cyclodextrin and sulfobutyl ether-β-cyclodextrin, or any combination thereof.

As preferable embodiment, the concentration of the water soluble cyclodextrin derivatives in the pharmaceutical formulation solution is in the range from 2%-60% (weight : volume ratio); and the concentration of gastrodin in the pharmaceutical formulation solution is in the range from 2%-60% (weight: volume ratio).

As further preferable embodiments, the concentration of the water soluble cyclodextrin derivatives in the pharmaceutical formulation solution is in the range from 3%-45% (weight : volume ratio); and the concentration of gastrodin in the pharmaceutical formulation solution is in the range from 3%-50% (weight: volume ratio).

As preferable embodiments, the concentration of PEG400 in the pharmaceutical formulation solution is in the range from 0.1%-20% (weight : volume ratio); and the concentration of mannitol in the pharmaceutical formulation solution is in the range from 1%-20% (weight: volume ratio).

As preferable embodiments, the microcirculation block includes block induced by brain microvascular injury and/or block induced by ischemical reperfusion injury and/or block induced by leucocyte adherence and/or block induced by peroxides.

As preferable embodiments, the preparation of the medicament for improving microcirculation block includes tablet, capsule, suppository, ointment, patch, pill, granule, suspension, injection, infusion solution or lyophilized powder.

Piceatannol-3'-o-β-d-glucopyranoside has applications in preparation of the medicaments for improving microcirculation block of mammals, including human, monkey, chimpanzee, baboon, cattle, horse, pig, sheep, dog, rabbit, rat, and mouse etc.

### Example 1:

| Formula | 1 | 2 | 3 | 4 | 5 |
|---|---|---|---|---|---|
| piceatannol-3'-o-β-d-glucopyranoside | 5g | 5g | 5g | 10g | 20g |
| Gastrodin | 0.5g | 80g | 400g | 80g | 200g |
| Sodium hydroxide or hydrochloric acid | q.s. | q.s. | q.s. | q.s. | q.s. |
| Water for injection | 1000 ml | 1000 ml | 1000 ml | 1000 ml | 1000 ml |

60% of water for injection was added to a tank based on the total amount of formulation, and heated to slight boiling at 95°C, which was subsequently maintained at 80°C. The solution was stirred for 10 min under vacuum. Subsequently, piceatannol-3'-o-β-d-glucopyranoside and gastrodin were added together to the tank at a prescribed amount by a flow of nitrogen. After about 5 min, piceatannol-3'-o-β-d-glucopyranoside was dissolved, and both vacuum and nitrogen were stopped. The pH value of the solution was adjusted to 5.0-7.0 and water was added until the total amount of formulation was achieved. The solution was then subjected to a series of routine operations including sterile filtration, filling, and lyophilisation to prepare an injection.

### Example 2:

| Formula | 1 | 2 |
|---|---|---|
| piceatannol-3'-o-β-d-glucopyranoside | 25 g | 50 g |
| Gastrodin | 80 g | 100 g |
| Hydroxypropyl-β-cyclodextrin | 30 g | 30 g |
| Sodium hydroxide or hydrochloric acid | q.s. | q.s. |
| Water for injection | 1000 ml | 1000 ml |

60% of water for injection was added to a tank based on the total amount of formulation, and heated to slight boiling at 95°C, which was then maintained at 80°C. The solution was stirred for 10 min under vacuum. Subsequently, piceatannol-3'-o-β-d-glucopyranoside and gastrodin as well as hydroxypropyl-β-cyclodextrin were added together to the tank at a prescribed amount by a flow of nitrogen. After about 5 min, piceatannol-3'-o-β-d-glucopyranoside was dissolved, and both vacuum and nitrogen were stopped. The pH value of the solution was adjusted to 6.0-7.0 and water was added until the total amount of formulation was achieved, i.e., the formulation procedure was completed. The solution was then subjected to a series of routine operations including sterile filtration, filling, and lyophilisation to prepare an injection.

### Reference Example 3:

| Formula | 1 | 2 | 3 | 4 |
|---|---|---|---|---|
| piceatannol-3'-o-β-d-glucopyranoside | 5 g | 10 g | 10 g | 20 g |
| Hydroxypropyl-β-cyclodextrin | 30 g | 30 g | 50 g | 50 g |
| Sodium hydroxide or hydrochloric acid | q.s. | q.s. | q.s. | q.s. |
| Water for injection | 1000 ml | 1000 ml | 1000 ml | 1000 ml |

60% of water for injection was added to a tank based on the total amount of formulation, and heated to slight boiling at 95°C, which was then maintained at 80°C. The solution was stirred for 10 min under vacuum. Subsequently, piceatannol-3'-o-β-d-glucopyranoside and hydroxypropyl-β-cyclodextrin were added together to the tank at a prescribed amount by a flow of nitrogen. After about 5 min, piceatannol-3'-o-β-d-glucopyranoside was dissolved, and both vacuum and nitrogen were stopped. The pH value of the solution was adjusted to 5.5-7.0 and water was added until the total amount of formulation was achieved, i.e., the formulation procedure was completed. The solution was then subjected to a series of routine operations including sterile filtration, filling, and lyophilisation to prepare an injection.

### Reference Example 4:

| Formula | 1 | 2 | 3 | 4 | 5 |
|---|---|---|---|---|---|
| piceatannol-3'-o-β-d-glucopyranoside | 10 g | 10 g | 10 g | 10 g | 10 g |
| Hydroxypropyl-β-cyclodextrin | 30 g | 30 g | 30 g | 30 g | 30 g |
| Antioxidant | Vitamin C 0.2 g | L-cysteine hydrochloride 0.2 g | Glutathione sodium 0.2 g | Sodium hydrogensulfite 0.2 g | EDTA2Na 0.2 g |
| Sodium hydroxide or hydrochloric acid | q.s. | q.s. | q.s. | q.s. | q.s. |
| Water for injection | 1000 ml | 1000 ml | 1000 ml | 1000 ml | 1000 ml |

5 parts of 60% of water for injection were each added to a tank based on the total amount of formulation, and heated to slight boiling at 95°C, which was then maintained at 80°C. The solution was stirred for 10 min under vacuum. Subsequently, piceatannol-3'-o-β-d-glucopyranoside and hydroxypropyl-β-cyclodextrin were added together to the tank at a prescribed amount by a flow of nitrogen. After about 5 min, piceatannol-3'-o-β-d-glucopyranoside was dissolved, and both vacuum and nitrogen were stopped. Vitamin C, L-cysteine hydrochloride, glutathione sodium, sodium bisulfite, and EDTA2Na were respectively added to the tank. The pH value of the solution was adjusted to 6.0-7.0 and water was added until the total amount of formulation was achieved, i.e., the formulation procedure was completed. The solution was then subjected to a series of routine operations including sterile filtration, and filling to prepare an injection, which was further used as the test solution. An injection free of the antioxidant was also prepared as the control solution using the same method. After 10% hydrogen peroxide solution was added and the solution was heated at 85°C for 60 h, it was found that the antioxidant-containing injection was colorless and clear, whereas the injection free of the antioxidant showed a yellow color, indicating that the injection can be stabilized by the antioxidant.

### Reference Example 5:

| Formula | piceatannol-3'-o-β-d-gluco pyranoside | PEG400 | Mannitol | Sodium hydroxide or hydrochloric acid | Water for injection |
|---|---|---|---|---|---|
| Usage | 5.0 g | 20 ml | 100 g | q.s. | 1000 ml |

60% of water for injection was added to a tank based on the total amount of formulation, and heated to slight boiling at 95°C, which was then maintained at 80°C. The solution was stirred for 10 min under vacuum. Subsequently, piceatannol-3'-o-β-d-glucopyranoside and PEG400 as well as mannitol were added together to the tank at a prescribed amount by a flow of nitrogen. After about 5 min, piceatannol-3'-o-β-d-glucopyranoside was dissolved, and both vacuum and nitrogen was stopped. The pH value of the solution was adjusted to 5.5-6.5 and water was added until the total amount of formulation was achieved, i.e., the formulation procedure was completed. The solution was then subjected to a series of routine operations including sterile filtration, filling, and lyophilisation to prepare an injection.

### Test example: solubility test

### 1. Methods

A series of solutions of gastrodin or hydroxypropyl-β-cyclodextrin or a mixture of gastrodin and hydroxypropyl-β-cyclodextrin were prepared in volumetric flasks according to the concentrations specified in the tables below. Excessive piceatannol-3'-o-β-d-glucopyranoside was added to the solutions prepared above, which were then shaken at 25±1°C in a thermostatic water bath continuously for 72 h until equilibrium was reached. The solutions were precisely taken out and filtered through a 0.45µm filter. The filtered solutions were diluted to prepare test solutions containing 6µg piceatannol-3'-o-β-d-glucopyranoside per milliliter. Appropriate amount of control sample was precisely weighed out, dissolved in water and diluted to prepare a control solution. The absorbance values of both types of solutions were measured at 319 nm using UV spectrophotometry, from which the solubility can be calculated. The test results of solubility can be seen in the tables below:

### 2. Solubilities of piceatannol-3'-o-β-d-glucopyranoside in the solutions containing various concentrations of gastrodin

| | | | | | | | |
|---|---|---|---|---|---|---|---|
| Gastrodin % | 0 | 1 | 3 | 5 | 8 | 10 | 15 |
| Piceatannol-3' -o-β-d-glucopyranoside (mg/ml) | 1.752 | 3.191 | 5.097 | 7.107 | 9.169 | 11.438 | 11.761 |
| Gastrodin % | 25 | 30 | 35 | 40 | 45 | 50 | |
| Piceatannol-3' -o-β-d-glucopyranoside (mg/ml) | 38.378 | 60.424 | 87.793 | 85.117 | 97.993 | 105.017 | |

### 3. Solubilities of piceatannol-3'-o-β-d-glucopyranoside in the solutions containing various concentrations of hydroxypropyl-β-cyclodextrin (Reference)

| | | | | | | | |
|---|---|---|---|---|---|---|---|
| Hydroxypropyl-β-cyctodextrin % | 0 | 1 | 3 | 5 | 8 | 10 | 15 |
| Piceatannol-3'-o-β-d-glucopyranoside (mg/ml) | 1.752 | 4.796 | 10.785 | 20.028 | 26.142 | 34.093 | 43.264 |
| Hydroxypropyl-β-cyclodextrin % | 20 | 25 | 30 | 35 | 40 | 45 | 50 |
| Piceatannol-3'-o-β-d-glucopyranoside (mg/ml) | 59.198 | 64.329 | 116.235 | 159.444 | 188.611 | 180.509 | 183.10 |

### 4. Solubilities of piceatannol-3'-o-β-d-glucopyranoside in the mixed solutions containing various concentrations of gastrodin and hydroxypropyl-β-cyclodextrin

| | | | | | | | |
|---|---|---|---|---|---|---|---|
| Gastrodin % | 0 | 1 | 3 | 5 | 8 | 10 | 15 |
| Hydroxypropyl-β-cyclodextrin % | 3 | 3 | 3 | 3 | 3 | 3 | 3 |
| Piceatannol-3'-o-β-d-glucopyranoside (mg/ml) | 10.785 | 14.786 | 16.215 | 22.61 | 30.945 | 39.811 | 55.987 |
| Gastrodin % | 20 | 25 | 30 | 35 | 40 | 45 | 50 |
| Hydroxypropyl-β-cyclodextrin % | 3 | 3 | 3 | 3 | 3 | 3 | 3 |
| Piceatannol-3'-o-β-d-glueopyranoside (mg/ml) | 107.193 | 126.647 | 181.272 | 233.379 | 197.257 | 186.789 | 188.029 |

### Experimental Example 1: The influence of piceatannol-3'-o-β-d-glucopyranoside of the present invention on the test microcirculation block in mice

### 1 Materials

### 1.1 Samples

The sample prepared in Example 5, specification: 10 mg/vial, No. 51# (Reference)
The sample prepared according to the formulation No. 3 in Example 3, specification: 20 mg/vial, No. 52# (Reference)
The sample prepared according to the formulation No. 2 in Example 1, specification: 10 mg/vial, No. 53#

All samples were white lyophilized preparations, easily soluble in water, and provided by Institute of Pharmaceutical Research, Kunming Pharmaceutical Corporation. Sample solutions were prepared at concentration of 1.0 mg/ml by sodium chloride injection (N.S) and administrated to mice by intravenous injection.

### 1.2 Reagents

Adrenalin hydrochloride injection, National Drug Approval Number: H41021054, 1 mg/ml, Tianjin Pharmaceutical Group Xinzheng CO., LTD Lot Number 0909102; sodium chloride injection (N.S), 500ml/bottle, National Drug Approval Number H53020723, Kunming Yusi Pharmaceutical CO., LTD, Lot Number 10062502; cedar oil for microscope, Shanghai Specimen and Model Factory, Lot Number 20101013.

Ethyl carbamate (Chemically pure, Beijing Chemical Reagent Company, Lot Number 20051007) was prepared to a 12% solution in sodium chloride injection for anaesthesia of mice.

### 1.3 Equipments

AL104 type electronic balance for sample measurement, Mettler-Toledo Instruments (Shanghai) Co., Ltd;
JJ2000 electronic balance for animal weight measurement, G & G measurement plant;
WX-6 microcirculation microscope, Chongqing Tianhai Medical Equipment Co., Ltd.

### 1.4 Animals

3- to 4-week old SPF grade Kunming mice, with half males and half females, weighing 18-22g, were obtained from Experimental Animal Center, Kunming Medical College under the production license: SCXK (Yunnan) 2011-0004.

The mice were raised in an IVC animal laboratory at temperature in the range from 20 to 25°C (daily temperature range ≤ 3°C), and humidity from 40% to 70%, with a 12:12 light : darkness cycle at illuminating value from 150 to 300 lx and with noise ≤ 60dB. The license for the use of experimental animal was SYXK (Yunan) 2005-0001.

Male and female mice were separately raised in group in transparent plastic boxes (≤ 10 rats in each box). Mice were daily fed with formula feedstuff and had free access to water. The box and pad were changed as appropriate. The production license for the feedstuff was: SCXK (Yunan) 2011-0005, and the feedstuff was provided by the Experimental Animal Center, Kunming Medical College.

### 1.5 Statistics

The results were expressed as mean ± standard deviation (*x̅* ±SD). LSD test was used for inter-group comparison for equal variance, and Dunnett's T3 test was used for inter-group comparison for unequal variance. Rank sum test was used for skewed distribution. A value of P < 0.05 was considered statistically significant, and a value of P < 0.01 was considered statistically highly significant.

### 2 Methods and results

### 2.1 Effect on the microcirculation of normal mice

40 mice (half males and half females) weighing 28-22 g were randomly divided in to 4 groups based on gender and body weight, including N.S control group; sample groups of 51 to 53# (10 mice in each group). After anaesthesia using 12% ethyl carbamate solution at a dosage of 10 ml/kg.bw by IP injection, the auricle was fixed, and the mouse was placed under a 100× microcirculation microscope. Appropriate capillary vessels (parallel artery and vein, the third stage branch) were selected as the observation object. When the conditions of animals were stable, base values were collected. Administration was given once by vena caudalis injection according to the dosage. For the control group, equal volume of N.S was given. All dosages were selected as 10 ml/kg.bw. Images were collected before administration and 5, 10, and 20 min after administration. 4 types of data were detected, including: (1) diameter of arteriole (A3); (2) diameter of venule (V3); (3) number of capillary vessel net; (4) flow state of blood. The variation rate of each data was calculated based on the base value collected before administration. The significance of inter-group difference was obtained by comparing with the solvent group. The room temperature was controlled at 22±2°C during experiment. Parallel operations were carried out to reduce systematic error. The results were listed in table 1-4.

As shown in Table 1-4, after administration of 10 ml/kg N.S by vena caudalis injection for the control group, there was no significant variation in the diameters of both arteriole and venule of auricle, the open number of capillary vessel net and the flow rate of blood. However, for the rest of groups, after administration, the diameters of both arteriole and venule of auricle increased to different extents, while there was no distinct effect on the open number of capillary vessel net and the flow rate of blood.

**Table 1. Effect on the diameter of arteriole of auricle of normal mice (x̅ ±SD, n=10)**

| Group | Dosage (/kg) | Base A3 diameter (µm) | Variation rate of A3 diameter after administration (%) | | |
|---|---|---|---|---|---|
| | | | 5 min | 10 min | 20 min |
| Control | 10 ml | 15.32±2.45 | 0.08±2.72 | 0.11±1.97 | 1.20±1.68 |
| 51# | 10 mg | 14.80±3.27 | 0.33±2.14 | 1.62±3.04 | 4.04±5.78 |
| 52# | 10 mg | 14.43±3.07 | 2.23±1.15* | 4.66±2.78** | 6.69±5.02** |
| 53# | 10 mg | 14.20±2.09 | 1.44±1.18 | 2.63±2.13* | 4.03±5.70 |

| | | | | | |
|---|---|---|---|---|---|
| As compared to the control group,*/**P<0.05/0.01; | | | | | |

**Table 2. Effect on the diameter of venule of auricle of normal mice (x̅ ±SD, n=10)**

| Group | Dosage (/kg) | Base V3 diameter (µm) | Variation rate of V3 diameter after administration (%)) | | |
|---|---|---|---|---|---|
| | | | 5 min | 10 min | 20 min |
| Control | 10 ml | 24.30±3.46 | -0.42±1.78 | 0.43±1.83 | 1.26±1.31 |
| 51# | 10 mg | 25.67±4.52 | 0.55±0.93 | 2.00±1.84 | 3.82±3.20* |
| 52# | 10 mg | 25.66±5.19 | 1.91±1.01** | 4.13±3.52** | 5.84±6.50* |
| 53# | 10 mg | 22.43±4.54 | 2.24±1.81** | 4.06±3.01** | 5.68±5.67* |

| | | | | | |
|---|---|---|---|---|---|
| As compared to the control group,*/**P<0.05/0.01; | | | | | |

**Table 3. Effect on the open number of capillary vessel net of auricle of normal mice (x̅ ±SD, n=10)**

| Group | Dosage (/kg) | Base value | Variation rate of the open number of capillary vessel net after administration (%)) | | |
|---|---|---|---|---|---|
| | | | 5 min | 10 min | 20 min |
| Control | 10 ml | 4.10±0.31 | 0.00±0.00 | 0.00±0.00 | 0.00±0.00 |
| 51# | 10 mg | 4.00±0.65 | 0.00±0.00 | 0.00±0.00 | 0.00±0.00 |
| 52# | 10 mg | 4.10±0.55 | 0.00±0.00 | 0.00±0.00 | 0.00±0.00 |
| 53# | 10 mg | 4.10±0.31 | 0.00±0.00 | 0.00±0.00 | 0.00±0.00 |

| | | | | | |
|---|---|---|---|---|---|
| As compared to the control group, P>0.05; | | | | | |

**Table 4. Effect on the flow state of blood of auricle of normal mice (x̅ ±SD, n=10)**

| Group | Dosage (/kg) | Base score | Variation rate of the score of blood flow state after administration (%)) | | |
|---|---|---|---|---|---|
| | | | 5 min | 10 min | 20 min |
| Control | 10 ml | 3.00±0.00 | 0.00±0.00 | 0.00±0.00 | 0.00±0.00 |
| 51# | 10 mg | 3.10±0.31 | 0.00±0.00 | 0.00±0.00 | 0.00±0.00 |
| 52# | 10 mg | 3.00±0.46 | 0.00±0.00 | 0.00±0.00 | 0.00±0.00 |
| 53# | 10 mg | 3.10±0.31 | 0.00±0.00 | 0.00±0.00 | 5.00±15.81 |

| | | | | | |
|---|---|---|---|---|---|
| As compared to the control group, P>0.05; | | | | | |

### 2.2 Effect on microcirculation block of model mice

After the images were collected 20 min after administration as mentioned above, experiment model of microcirculation block was created by IP injection of adrenalin hydrochloride to the mouse at a dosage of 0.2 mg/kg. For the control group, equal volume of N.S was given. All dosages were selected as 10 ml/kg.bw. Images were consecutively collected 5, 10, 20, and 30 min after model established. The variation rate of each data after model establishment was calculated using the value detected 20 min after administration as the base value. The significance of inter-group difference was obtained by comparing with the solvent group. The type of data detected and the experiment conditions were the same as the above mentioned. The results were shown in Table 5-8.

It can be seen in Table 5-8 that after IP injection of 0.2 mg/kg.bw adrenalin hydrochloride to the model mice, distinct shrinkage of arteriole and venule of auricle can be observed, the open number of capillary vessel net was significantly reduced, and the blood flow rate was slower, which indicated the successful creation of the microcirculation block model. While for the rest of groups, it can be seen that the medicament can evidently antagonize the shrinkage of arteriole and venule of model mice with microcirculation block and the decrease of the open number of capillary vessel net resulted from adrenalin, and the blood flow slowing.

**Table 5. Effect on the diameter of arteriole of auricle of model mice with microcirculation block (x̅ ±SD, n=10)**

| Group | Dosage (/kg) | Base A3 diamter (µm) | Variation rate of A3 diameter after model creation (%)) | | | |
|---|---|---|---|---|---|---|
| | | | 5 min | 10 min | 20 min | 30 min |
| Model | 0.2 mg | 15.32±2.45 | -7.01±2.74 | -10.33±2.61 | -13.33±2.01 | -15.60±1.47 |
| 51# | 10 mg | 14.80±3.27 | -3.87±3.64* | -8.30±3.55 | -5.80±3.51** | -1.08±3.93** |
| 52# | 10 mg | 14.43±3.07 | -0.09±5.00** | -3.00±5.41** | -1.35±6.23** | 1.83±7.22** |
| 53# | 10 mg | 14.20±2.09 | -2.15±4.74* | -5.56±5.28* | -3.05±6.30** | 1.48±6.57** |

| | | | | | | |
|---|---|---|---|---|---|---|
| As compared to the model group, */**P<0.05/0.01 | | | | | | |

**Table 6. Effect on the diameter of venule of auricle of model mice with microcirculation block (x̅ ±SD, n=10)**

| Group | Dosage (/kg) | Base V3 diamter (µm) | Variation rate of V3 diameter after model creation (%)) | | | |
|---|---|---|---|---|---|---|
| | | | 5 min | 10 min | 20 min | 30 min |
| Model | 0.2mg | 24.30±3.46 | -6.48±2.70 | -10.65±3.23 | -13.47±3.27 | -15.72±2.91 |
| 51# | 10mg | 25.67±4.52 | -4.31±3.14 | -7.19±3.02* | -5.88±3.27** | -3.68±3.42** |
| 52# | 10mg | 25.66±5.19 | -1.90±4.43* | -4.32±5.28** | -3.29±6.79** | -0.35±7.56** |
| 53# | 10mg | 22.43±4.54 | -3.23±5.80 | -6.30±5.35* | -3.84±5.58** | -0.74±6.85** |

| | | | | | | |
|---|---|---|---|---|---|---|
| As compared to the model group, */**P<0.05/0.01 | | | | | | |

**Table 7. Effect on the open number of capillary vessel net of auricle of model mice with microcirculation block (x̅ ±SD, n=10)**

| Group | Dosage (/kg) | Base value | Variation rate of the open number of capillary vessel net after model creation (%)) | | | |
|---|---|---|---|---|---|---|
| | | | 5 min | 10 min | 20 min | 30 min |
| Mode 1 | 0.2 mg | 4.10±0.31 | -46.50±8.18 | -61.00±12.43 | -61.00±12.43 | -58.50±11.80 |
| 51# | 10 mg | 4.00±0.65 | -39.17±11.82 | -44.17±9.66** | -41.67±11.11** | -41.67±11.11** |
| 52# | 10 mg | 4.10±0.55 | -34.67±10.02* | -44.67±7.24** | -42.17±9.23** | -40.17±11.61** |
| 53# | 10 mg | 4.10±0.31 | -31.83±11.07** | -46.33±6.18** | -36.83±12.56** | -34.33±12.13** |

| | | | | | | |
|---|---|---|---|---|---|---|
| As compared to the model group, */**P<0.05/0.01 | | | | | | |

**Table 8. Effect on the blood flow state of auricle of model mice with microcirculation block (x̅ ±SD, n=10)**

| Group | Dosage (/kg) | Base score | Variation rate of the score of blood flow state after model creation (%)) | | | |
|---|---|---|---|---|---|---|
| | | | 5 min | 10 min | 20 min | 30 min |
| Mode 1 | 0.2mg | 4.10±0.31 | -29.17±10.58 | -31.67±12.30 | -35.00±16.57 | -31.67±12.30 |
| 51# | 10mg | 4.00±0.65 | -10.00±16.10** | -20.00±17.21 | -23.33±16.10 | -23.33±16.10 |
| 52# | 10mg | 4.10±0.55 | -9.17±14.93** | -15.83±16.87* | -19.17±16.69* | -19.17±16.69 |
| 53# | 10mg | 4.10±0.31 | -7.50±25.59* | -7.50±25.59* | -10.83±26.66* | -10.83±26.66* |

| | | | | | | |
|---|---|---|---|---|---|---|
| As compared to the model group, */**P<0.05/0.01 | | | | | | |

### 3. Conclusions

3.1 Expansion effect on capillaries of the normal mice can be seen after single administration of all samples by intravenous injection;
3.2 After single administration of all samples by intravenous injection, the medicaments can evidently antagonize the shrinkage of arteriole and venule and the reduction of the open number of capillary vessel net of the model mice with microcirculation block resulted from adrenalin, and can obviously confront the blood flow slowing, indicating that all medicaments were effective for the improvement of microcirculation block.

### Experimental Example 2: The effect of piceatannol-3'-o-β-d-glucopyranoside of the present invention on burn shock of SD rat

1. 40 SPF grade SD rats (half males and half females), weighing 300-360 g, were provided by Experimental Animal Center, Kunming Medical College.

### 2. Experimental methods:

SD rats (half males and half females) were randomly divided in to groups including N.S control group and sample groups of 51 to 53# (10 mice in each group). After anaesthesia by ethyl carbamate-ketamine, 30% surface area of the part below waist was scalded by 80°C water for 30 sec. Intravenous injection was given to each group 0.5 h after scalded: 10ml N.S for the control group, and 10 ml sample for the sample group 51# to 53#.

### 3. Conclusion:

The survival time after burn shock of SD rats can be significantly extended after single intravenous injection of all samples.

**Table 9. Effect on the burn shock of rats (x̅ ±SD, n=10)**

| Group | Dosage (/kg) | Survival time (hr) |
|---|---|---|
| Model group | 10ml | 7.0±2.6 |
| 51# | 10mg | 14.5±3.2** |
| 52# | 10mg | 16.1±2.9** |
| 53# | 10mg | 17.6±3.4** |

| | | |
|---|---|---|
| As compared to the model group, */**P<0.05/0.01 | | |

## Claims

1. A pharmaceutical formulation solution comprising piceatannol-3'-o-β-d-glucopyranoside and gastrodin for use in improving microcirculation block,
wherein the piceatannol-3'-o-β-d-glucopyranoside is (E)-1-(3,5-dihydroxyphenyl)-2-(3-hydroxy-4-O-β-D-glucopyranose phenyl)ethylene of the following structure: 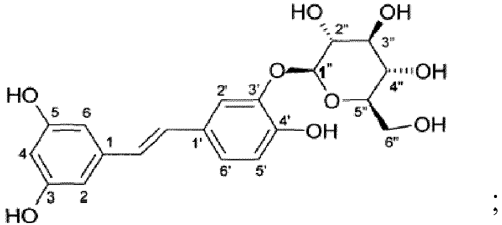 wherein the microcirculation block is selected from block induced by brain microvascular injury and/or block induced by ischemical reperfusion injury.

2. The pharmaceutical formulation solution for use in improving microcirculation block according to claim 1, which is **characterized in that**: the content of piceatannol-3'-o-β-d-glucopyranoside in the pharmaceutical formulation solution is in the range from 1.23%-90.9% by weight.

3. The pharmaceutical formulation solution for use in improving microcirculation block according to claim 1, further comprising water soluble cyclodextrin derivatives, selected from methyl-β-cyclodextrin, hydroxypropyl-β-cyclodextrin, thioether-β-cyclodextrin and sulfobutyl ether-β-cyclodextrin, or any combination thereof.

4. The pharmaceutical formulation solution for use in improving microcirculation block according to claim 3, wherein the concentration of the water soluble cyclodextrin derivatives in the pharmaceutical formulation solution is in the range from 2%-60% (weight : volume ratio); and the concentration of gastrodin in the pharmaceutical formulation solution is in the range from 2%-60% (weight: volume ratio).

5. The pharmaceutical formulation solution for use in improving microcirculation block according to claim 4, which is **characterized in that**: the concentration of the water soluble cyclodextrin derivatives in the pharmaceutical formulation solution is in the range from 3%-45% (weight: volume ratio); and the concentration of gastrodin in the pharmaceutical formulation solution is in the range from 3%-50% (weight: volume ratio).

## Patentansprüche

1. Pharmazeutische Formulierungslösung, umfassend Piceatannol-3'-o-β-d-glucopyranosid und Gastrodin zur Verwendung bei der Verbesserung von Mikrozirkulationsblockade,
wobei das Piceatannol-3'-o-β-d-glucopyranosid (E)-1-(3,5-Dihydroxyphenyl)-2-(3-hydroxy-4-O-β-D-glucopyranosephenyl)ethylen der folgenden Struktur ist: 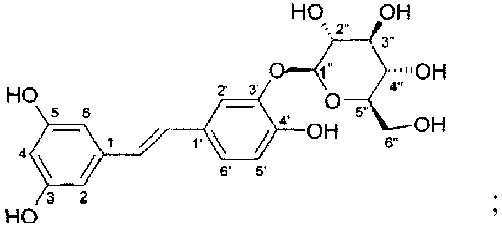 wobei die Mikrozirkulationsblockade aus Blockade induziert durch mikrovaskuläre Hirnverletzung und/oder Blockade induziert durch ischämische Reperfusionsverletzung ausgewählt ist.

2. Pharmazeutische Formulierungslösung zur Verwendung bei der Verbesserung von Mikrozirkulationsblockade nach Anspruch 1, die **dadurch gekennzeichnet ist, dass**: der Gehalt an Piceatannol-3'-o-β-d-glucopyranosid in der pharmazeutischen Formulierungslösung in der Spanne von 1,23 Gew.-%-90,9 Gew.-% liegt.

3. Pharmazeutische Formulierungslösung zur Verwendung bei der Verbesserung von Mikrozirkulationsblockade nach Anspruch 1, ferner umfassend wasserlösliche Cyclodextrinderivate, ausgewählt aus Methyl-β-cyclodextrin, Hydroxypropyl-β-cyclodextrin, Thioether-β-cyclodextrin und Sulfobutylether-β-cyclodextrin oder einer beliebigen Kombination davon.

4. Pharmazeutische Formulierungslösung zur Verwendung bei der Verbesserung von Mikrozirkulationsblockade nach Anspruch 3, wobei die Konzentration der wasserlöslichen Cyclodextrinderivate in der pharmazeutischen Formulierungslösung in der Spanne von 2 %-60 % (Verhältnis Gewicht:Volumen) ist; und die Konzentration von Gastrodin in der pharmazeutischen Formulierungslösung in der Spanne von 2 %-60 % (Verhältnis Gewicht:Volumen) ist.

5. Pharmazeutische Formulierungslösung zur Verwendung bei der Verbesserung von Mikrozirkulationsblockade nach Anspruch 4, die **dadurch gekennzeichnet ist, dass**: die Konzentration der wasserlöslichen Cyclodextrinderivate in der pharmazeutischen Formulierungslösung in der Spanne von 3 %-45 % (Verhältnis Gewicht:Volumen) ist; und die Konzentration von Gastrodin in der pharmazeutischen Formulierungslösung in der Spanne von 3 %-50 % (Verhältnis Gewicht:Volumen) ist.

## Revendications

1. Solution de formulation pharmaceutique comprenant du piceatannol-3'-o-β-d-glucopyranoside et de la gastrodine destinée à être utilisé dans l'amélioration d'un blocage de la microcirculation, dans laquelle le piceatannol-3'-o-β-d-glucopyranoside est le (E)-1-(3,5-dihydroxyphényl)-2-(3-hydroxy-4-O-β-D-glucopyranose phényl)éthylène de la structure suivante : 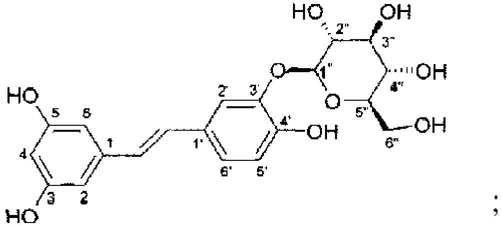 dans laquelle le blocage de la microcirculation est sélectionné parmi le blocage induit par une lésion microvasculaire cérébrale et/ou le blocage induit par une lésion de reperfusion ischémique.

2. Solution de formulation pharmaceutique destinée à être utilisée dans l'amélioration d'un blocage de la microcirculation selon la revendication 1, qui est **caractérisée en ce que** : la teneur en piceatannol-3'-o-β-d-glucopyranoside dans la solution de formulation pharmaceutique est dans la plage de 1,23 % en poids à 90,9 % en poids.

3. Solution de formulation pharmaceutique destinée à être utilisée dans l'amélioration d'un blocage de la microcirculation selon la revendication 1, comprenant en outre des dérivés de la cyclodextrine solubles dans l'eau, sélectionnés parmi la méthyl-β-cyclodextrine, l'hydroxypropyl-β-cyclodextrine, la thioéther-β-cyclodextrine et la sulfobutyl éther-β-cyclodextrine, ou toute combinaison de ceux-ci.

4. Solution de formulation pharmaceutique destinée à être utilisée dans l'amélioration d'un blocage de la microcirculation selon la revendication 3, dans laquelle la concentration des dérivés de la cyclodextrine solubles dans l'eau dans la solution de formulation pharmaceutique est dans la plage de 2 % à 60 % (rapport poids/volume) ; et la concentration de la gastrodine dans la solution de formulation pharmaceutique est dans la plage de 2 % à 60 % (rapport poids/volume).

5. Solution de formulation pharmaceutique destinée à être utilisée dans l'amélioration d'un blocage de la microcirculation selon la revendication 4, qui est **caractérisée en ce que** : la concentration des dérivés de la cyclodextrine solubles dans l'eau dans la solution de formulation pharmaceutique est dans la plage de 3 % à 45 % (rapport poids/volume) ; et la concentration de la gastrodine dans la solution de formulation pharmaceutique est dans la plage de 3 % à 50 % (rapport poids/volume).
